# EUROPEAN PATENT APPLICATION

(11) **EP 1 698 277 A1**
(43) Date of publication of application: **06.09.2006**
(21) Application number: 06004397.3
(22) Date of filing: 03.03.2006
(51) Int. Cl.: A61B 5/053, G01G 19/50

(54) **Biological data measurement apparatus**

(30) Priority: 04.03.2005 JP 2005061123; 31.10.2005 JP 2005315816
(71) Applicant: TANITA CORPORATION, Tokyo 174-0063 (JP)
(72) Inventor: Miyashita, Yuichi, Itabashi-ku Tokyo 174-0063 (JP); Kobayashi, Kotaku, Itabashi-ku Tokyo 174-0063 (JP); Kumagai, Takashi, Itabashi-ku Tokyo 174-0063 (JP); Ishikawa, Toshihiko, Illinois 60005 (US); Montagnino, James G., St. Charles Illinois 60174 (US)
(74) Representative: Müller-Boré & Partner Patentanwälte

(57) **Abstract**

Disclosed a biological data measurement apparatus, which includes data measurement means for measuring plural kinds of biological data about a user, data selection means for selecting one of the plural kinds of measured biological data, and data display means for displaying the selected biological datum. The biological data measurement apparatus comprises a plurality of light emitters corresponding, respectively, to the plural kinds of biological data, and lighting control means for activating one of the light emitters which corresponds to one selected from the biological data by the data selection means. The biological data measurement apparatus of the present invention can eliminate the risk of causing user's confusion about biological data display and operational complication, at the lowest possible cost.

## Description

### TECHNICAL FIELD

The present invention relates to a biological data measurement apparatus for measuring biological data about a user, and more specifically to a biological data measurement apparatus including data measurement means for measuring plural kinds of biological data about a user, data selection means for selecting one of the plural kinds of measured biological data, and data display means for displaying the selected biological datum.

### BACKGROUND ART

There has been widely known an apparatus for measuring plural kinds of biological data about a user, such as body weight and body fat percentage (see, for example, the following Patent Publication 1). Particularly, in recent biological data measurement apparatuses, the number of kinds or items of measurable biological data has been constantly increased to allow a user to measure various biological data, such as visceral fat area, body water percentage, muscle mass, bone mass and basal metabolic rate, as well as body weight and body fat percentage.

As the result of increase in the number of kinds of measurable biological data, data display means mounted on a biological data measurement apparatus, such as a liquid-crystal display screen, is apt to have difficulty in displaying the entire measurement results thereon at a time. For this reason, there have been developed a biological data measurement apparatus designed to automatically select and display each of plural kinds of measured biological data one-by-one at given time intervals, and a biological data measurement apparatus designed to display each of plural kinds of measured biological data one-by-one according to each selection operation performed by a user. In particular, the latter biological data measurement apparatus includes one type which is provided with a plurality of key switches corresponding, respectively, with measurement items, and designed to allow a user to selectively press any one of the key switches so as to display one biological datum corresponding to the pressed key switch (see, for example, the following Patent Publication 2). That is, this type of biological data measurement apparatus is designed to allow a user to directly select a biological datum to be displayed.
Patent Publication 1: Japanese Patent Publication No. 05-049050
Patent Publication 2: Japanese Patent Application No. 2004-048406

### DISCLOSURE OF INVENTION

Among the conventional biological data measurement apparatuses capable of measuring plural kinds of biological data, the type designed to automatically select (switch) and display each of plural kinds of measurement results one-by-one is likely to have a problem about difficulty in finding out what a biological datum is displayed moment-to-moment, when the frequency of display switchings is increased along with increase in the number of kinds of biological data. Specifically, most of the biological data measurement apparatuses are designed to display only a numerical value and unit of each biological datum or measurement result on the liquid-crystal display screen. Thus, if plural kinds of biological data having the same unit, such as body weight (kg), body fat mass (kg), muscle mass (kg) and bone mass (kg), are successively displayed, a user is likely to fall into confusion about "which of the biological data a currently displayed value indicates". This confusion can be tentatively solved by displaying a character or mark representing each biological datum together with the biological datum on the liquid-crystal display screen, and such a data display technique is actually employed in some commercial biological data measurement apparatuses. However, from a practical standpoint, the liquid-crystal display screen is limited in space allocatable to such characters or marks, and this solution inevitably leads to increase in size of the liquid-crystal display screen itself. This is likely to cause a problem about increase in total cost of the apparatus.

Differently from the above type, in the biological data measurement apparatus of the type designed to display each of plural kinds of measurement results one-by-one according to each selection operation performed by a user, the user can freely switch and select a biological datum to be displayed, and thereby the above confusion hardly occurs. In reality, if the biological data measurement apparatus has only one or two key switches for selecting (switching) the display item, a user is required to perform a selection operation while taking account of "how many times the key switch has to be pressed to reach a desired biological datum" or "how many times the key switch has been pressed until now", and the operation itself inevitably becomes complicated. While the biological data measurement apparatus may be designed to have a plurality of key switches corresponding to all measurement items so as to allow a user to directly select a biological datum to be displayed, the key switches having the same number as that of kinds of the biological data will be arranged on a casing of the apparatus, and thereby a user has to select one of the key switches corresponding to a desired biological datum. When the number of kinds of the biological data is no more than three or four, this operation would not cause any problem. However, if the number is gradually increased to five or six, and further to seven, eight, nine ---, the key-switch selection itself will become significantly complicated, which is liable to cause user's confusion.

In view of the above problems in the conventional biological data measurement apparatus including data measurement means for measuring plural kinds of biological data about a user, data selection means for selecting one of the plural kinds of measured biological data, and data display means for displaying the selected biological datum, it is therefore an object of the present invention to provide a biological data measurement apparatus capable of eliminating the aforementioned risk of causing user's confusion about biological data display and the operational complication, at the lowest possible cost.

In order to achieve this object, the present invention provides a biological data measurement apparatus including data measurement means for measuring plural kinds of biological data about a user, data selection means for selecting one of the plural kinds of measured biological data, and data display means for displaying the selected biological datum, which is characterized by comprising a plurality of light emitters corresponding, respectively, to the plural kinds of biological data, and lighting control means for activating one of the light emitters which corresponds to one selected from the biological data by the data selection means.

In the biological data measurement apparatus of the present invention, each of the light emitters may include a light-transparent film covering thereover and having a character or mark distinctively representing each biological datum associated therewith.

In the biological data measurement apparatus of the present invention, the data selection means may include automatic data-selection means for automatically selecting each of the plural kinds of biological data one-by-one at given time intervals, and arbitrary data-selection means for arbitrarily selecting one of the plural kinds of biological data according to a selection operation performed by the user. In this case, the lighting control means may be operable to change a lighting mode of the light emitters depending on whether the selection of the biological data is performed by the automatic data-selection means or the arbitrary data-selection means.

The biological data measurement apparatus of the present invention may further comprise data storage means for storing the plural kinds of biological data measured by the data measurement means. Further, the data selection means may include latest-data selection means for selecting one of latest biological data newly measured by the data measurement means, and past-data selection means for selecting one of past biological data stored on the data storage means. In this case, the lighting control means may be operable to change a lighting mode of the light emitters depending on whether the selection of the biological data is performed by the latest-data selection means or the past-data selection means.

In the biological data measurement apparatus of the present invention, the data selection means may include a plurality of key switches associated, respectively, with the plural kinds of biological data, and each of the plurality of light emitters is incorporated in one of the key switches which is associated with the biological datum corresponding to the light emitter.

In the biological data measurement apparatus of the present invention, the data measurement means may include an input key for inputting at least a body height datum of the user, a weight sensor for measuring a body weight datum of the user, and a combination of an electrode and an electric circuit, for measuring a datum about an impedance between the feet of the user. In this case, the plural kinds of biological data may consist of a plurality of index values about a body composition of the user to be calculated based on at least the body height, body weight and impedance data.

According to the biological data measurement apparatus of the present invention, when one biological datum selected by the data selection means is displayed on the data display means, one light emitter associated with the selected biological datum is activated by the lighting control means. This allows the user to readily recognize the kind of the currently displayed biological datum on the data display means, without any confusion. In addition, the use of these light emitters makes it possible to completely omit or otherwise simplify the display of characters or marks representing the biological data in the data display means, so as to avoid the problem about increase in size of the data display means and the resulting problem about increase in total cost of the biological data measurement apparatus.

As one of measures for clarifying the correspondence between the light emitters and the biological data in the biological data measurement apparatus of the present invention, a character or mark may be labeled or formed on the vicinity of each of the corresponding light emitters. In particular, each of the light emitters may be covered with the light-transparent film having a character or mark distinctively representing each biological datum associated therewith to provide excellent visibility and enhanced entire appearance of the apparatus.

Further, when the data selection means in the biological data measurement apparatus of the present invention is designed to select a biological datum to be displayed on the data display means by use of both the automatic data-selection means for automatically selecting each of the biological data one-by-one at given time intervals and the arbitrary data-selection means for arbitrarily selecting each of the biological data according to each selection operation performed by the user, the lighting mode of the light emitters is changed depending on the respective cases. This makes it possible to allow the user to readily recognize the difference in current display state, such as whether the display switching is performed in an automatic manner or in a manual manner requiring a user's operation, so as to provide enhanced user-friendliness.

When the biological data measurement apparatus of the present invention is further provided with the data storage means for storing the plural kinds of biological data measured by the data measurement means, and designed to select a biological datum to be displayed on the data display means by use of both the latest-data selection means for selecting one of the latest biological data and the past-data selection means for selecting one of the past biological data, the lighting mode of the light emitters is changed depending on the respective cases. This makes it possible to allow the user to readily recognize the difference in current display state, such as whether the data display means displays a newly measured biological datum or a previously measured biological data, so as to provide enhanced user-friendliness.

When the biological data measurement apparatus of the present invention is designed such that the data selection means includes the plurality of key switches associated, respectively, with the plural kinds of biological data, and each of the plurality of light emitters is incorporated in one of the key switches which is associated with the biological datum corresponding to the light emitter, one of the biological data selected by using one of the key switches serving as the data selection means is displayed on the data display means, and simultaneously one of the light emitters which corresponds to the selected biological datum and incorporated in the key switch used in the selection is activated by the lighting control means. Thus, light can be emitted from the key switch used in the selection to allow the user to directly/intuitively recognize the kind of the biological datum currently displayed on the data display means.

The data measurement means in the biological data measurement apparatus of the present invention may be composed of an input key for inputting at least a body height datum of the user, a weight sensor for measuring a body weight datum of the user and a combination of an electrode and an electric circuit for measuring a datum about an impedance between the feet of the user, so as to measure the plural kinds of measured biological data which can serve as a plurality of index values about a body composition of the user to be calculated based on at least the body height, body weight and impedance data. In this way, the biological data measurement apparatus of the present invention can be achieved as an apparatus for measuring a body composition of a user.

### BRIEF DESCRIPTION OF DRAWINGS

FIG 1 is a perspective external view of a biological data measurement apparatus 1 (1') according to a first (second) embodiment of the present invention.
FIG 2 is a schematic block diagram of an electric circuit configuration incorporated in the biological data measurement apparatus 1 (1').
FIG 3 is a flowchart of a personal-data registration process to be performed in the biological data measurement apparatus 1 (1').
FIG 4 is a flowchart of a biological-data measurement process to be performed in the biological data measurement apparatus 1 (1').
FIG. 5 is a flowchart of a latest-biological-data display process to be performed in the biological data measurement apparatus 1 according to the first embodiment.
FIG 6 is a flowchart of a past-biological-data display process to be performed in the biological data measurement apparatus 1 according to the first embodiment.
FIGS. 7(A) to 7(D) are explanatory diagrams of various examples of a display of a liquid-crystal display screen and a lighting mode of light emitters in the biological data measurement apparatus 1 according to the first embodiment.
FIG 8 is a flowchart of a latest-biological-data display process to be performed in the biological data measurement apparatus 1' according to the second embodiment.
FIG 9 is a flowchart of a latest-biological-data display process to be performed in the biological data measurement apparatus 1' according to the second embodiment.
FIGS. 10(A) to 10(D) are explanatory diagrams of various examples of a display of a liquid-crystal display screen and a lighting mode of light emitters in the biological data measurement apparatus 1' according to the second embodiment.

### BEST MODE FOR CARRYING OUT THE INVENTION

A biological data measurement apparatus of the present invention includes data measurement means for measuring plural kinds of biological data about a user, data selection means for selecting one of the plural kinds of measured biological data, data display means for displaying the selected biological datum, a plurality of light emitters corresponding, respectively, to the plural kinds of biological data, and lighting control means for activating one of the light emitters which corresponds to one selected from the biological data by the data selection means.

Preferably, each of the light emitters includes a light-transparent film covering thereover and having a character or mark distinctively representing each biological datum associated therewith.

In the biological data measurement apparatus of the present invention, it is preferable that the data selection means includes automatic data-selection means for automatically selecting each of the plural kinds of biological data one-by-one at given time intervals, and arbitrary data-selection means for arbitrarily selecting one of the plural kinds of biological data according to a selection operation performed by the user, wherein the lighting control means is operable to change a lighting mode of the light emitters depending on whether the selection of the biological data is performed by the automatic data-selection means or the arbitrary data-selection means.

Preferably, the biological data measurement apparatus of the present invention further comprises data storage means for storing the plural kinds of biological data measured by the data measurement means, wherein the data selection means includes latest-data selection means for selecting one of latest biological data newly measured by the data measurement means, and past-data selection means for selecting one of past biological data stored on the data storage means, and wherein the lighting control means is operable to change a lighting mode of the light emitters depending on whether the selection of the biological data is performed by the latest-data selection means or the past-data selection means.

In the biological data measurement apparatus of the present invention, it is preferable that the data selection means includes a plurality of key switches associated, respectively, with the plural kinds of biological data, and each of the plurality of light emitters is incorporated in one of the key switches which is associated with the biological datum corresponding to the light emitter.

In the biological data measurement apparatus of the present invention, it is preferable that the data measurement means includes an input key for entering at least a body height datum of the user, a weight sensor for measuring a body weight datum of the user, and a combination of an electrode and an electric circuit, for measuring a datum about an impedance between the feet of the user, wherein the plural kinds of biological data consist of a plurality of index values about a body composition of the user to be calculated based on at least the body height, body weight and impedance data.

### [FIRST EMBODIMENT]

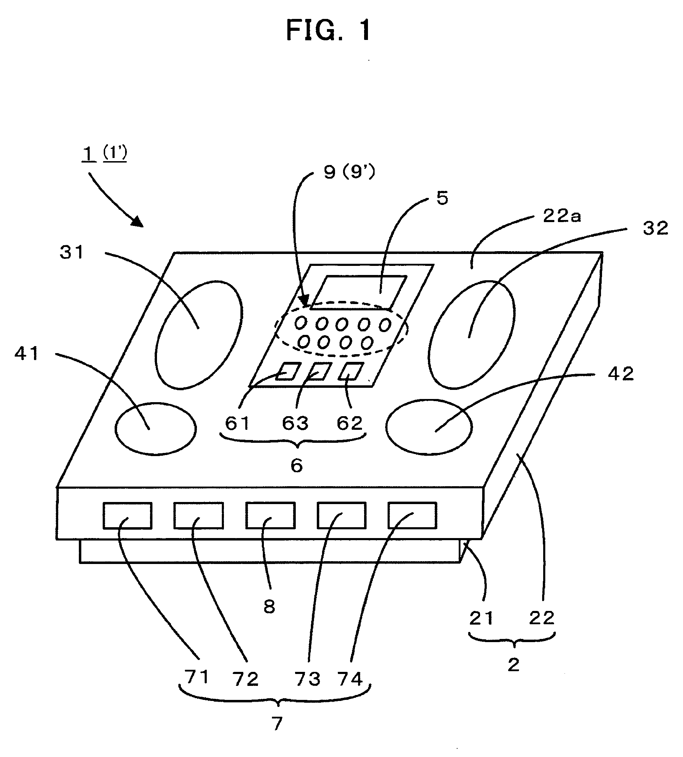

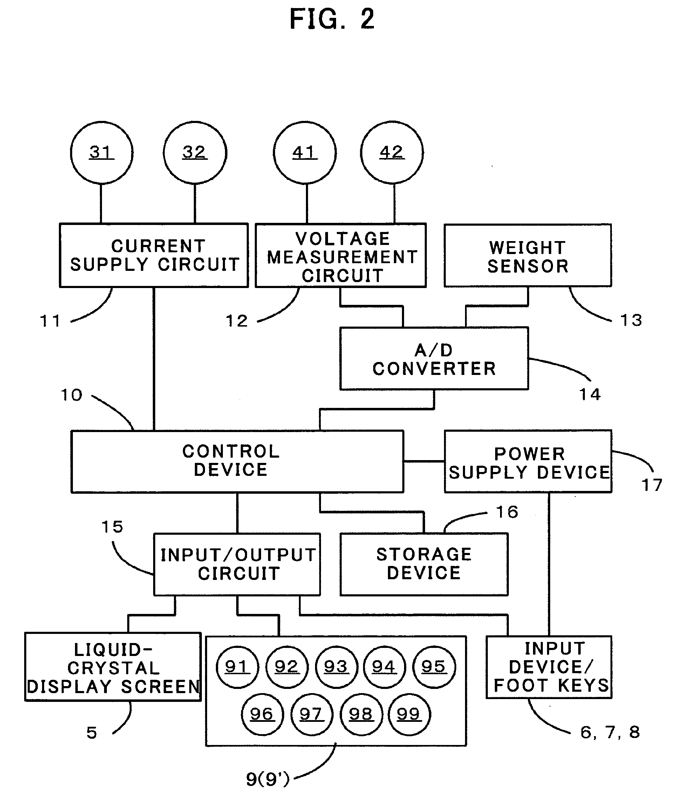

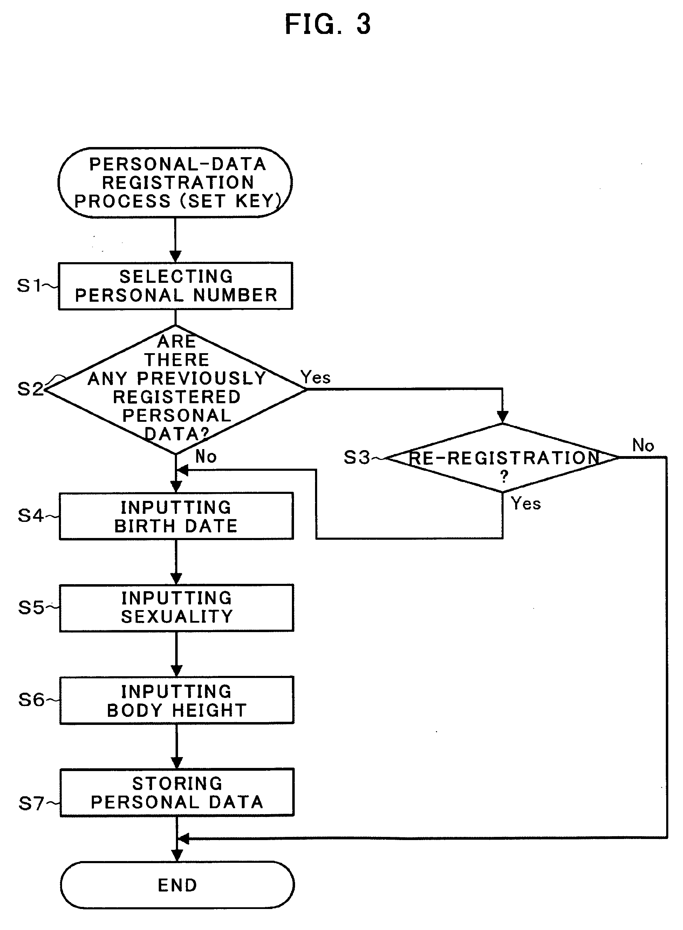

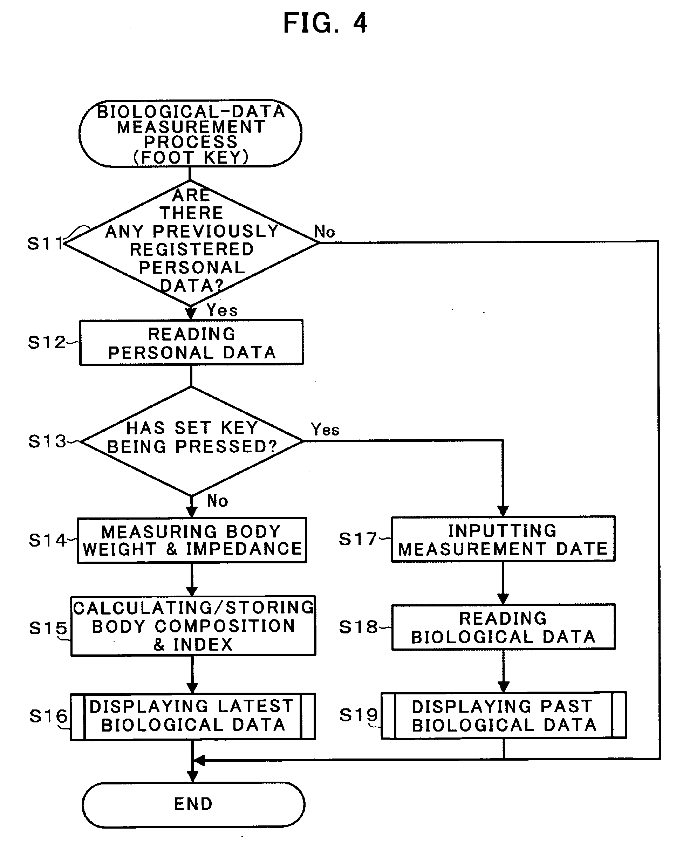

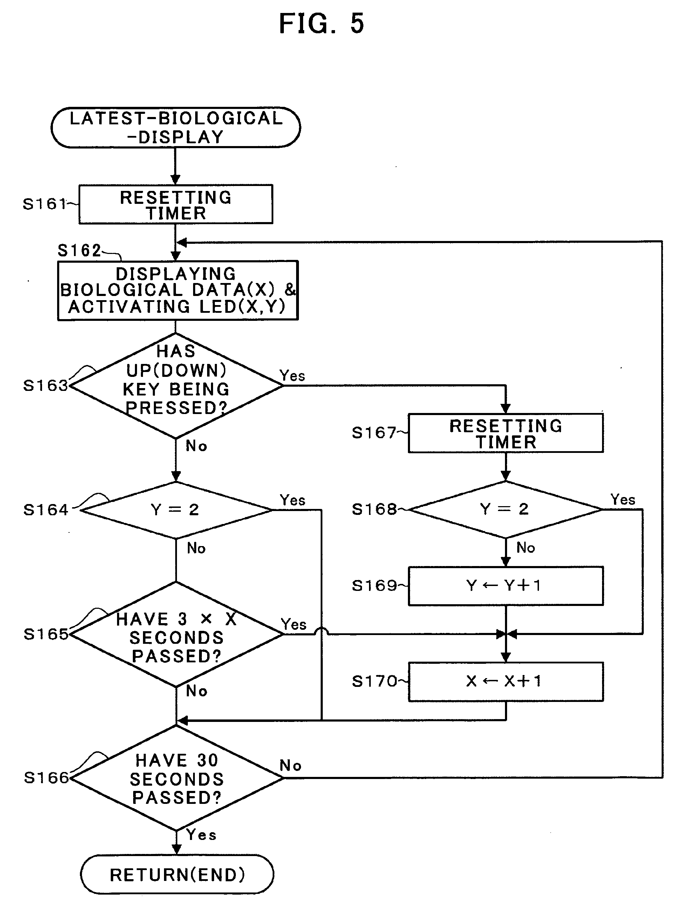

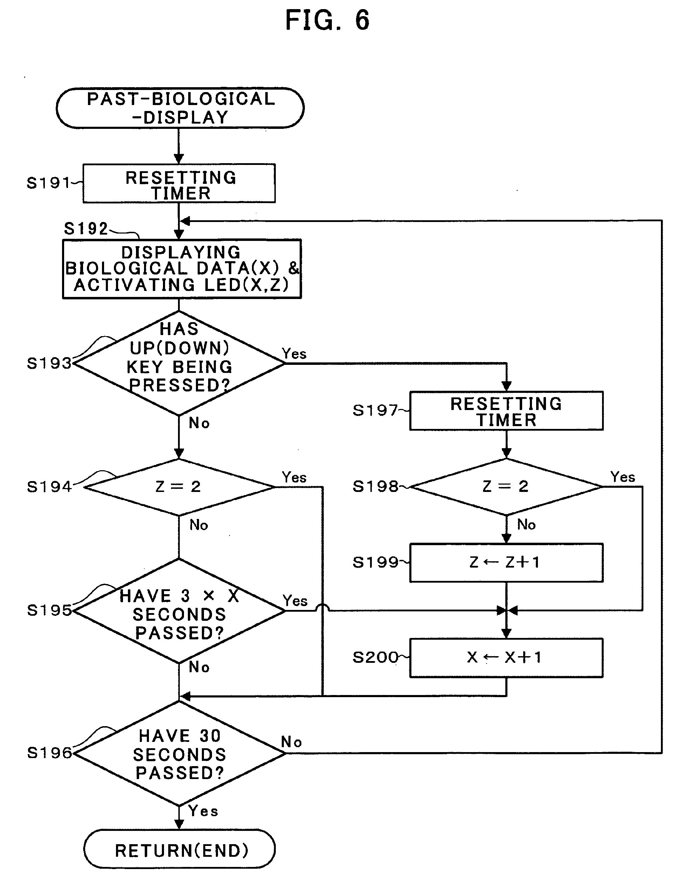

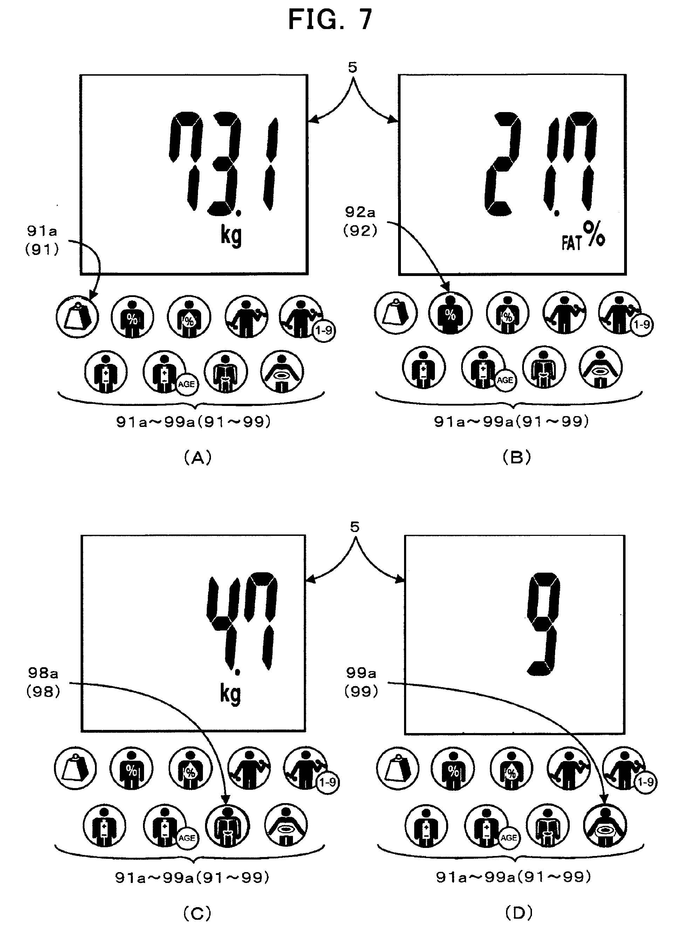

With reference to the drawings, a first preferred embodiment of the present invention will now be described. FIG 1 is a perspective external view of a biological data measurement apparatus 1 according to the first embodiment, and FIG 2 is a schematic block diagram of an electric circuit configuration incorporated in the biological data measurement apparatus 1. FIG 3 is a flowchart of a personal-data registration process to be executed in the biological data measurement apparatus 1, and FIG 4 is a flowchart of a biological-data measurement process to be executed in the biological data measurement apparatus 1. FIG. 5 is a flowchart of a latest-biological-data display process to be executed in the biological data measurement apparatus 1, and FIG 6 is a flowchart of a past-biological-data display process to be executed in the biological data measurement apparatus 1. FIGS. 7(A) to 7(D) are explanatory diagrams of various examples of a display of a liquid-crystal display screen and a lighting mode of light emitters, in the biological data measurement apparatus 1.

This biological data measurement apparatus 1 (hereinafter referred to simply as "apparatus 1") is an improved type of the so-called "body composition analyzer" designed to calculate body composition data about a user, such as body fat percentage, body water percentage, muscle mass, basal metabolic rate or bone mass, by use of user's data about age, sexuality, body height, body weight and inter-foot impedance (bioelectric impedance between feet). Specifically, based on the above user's data, the apparatus 1 is designed to additionally calculate a biological index datum, such as visceral fat level, physique level or metabolic age. In the following description about this embodiment, the above age, sexuality and body height data will hereinafter be referred to collectively as "personal data", and the total nine data consisting of the body weight datum, the five body composition data and the three biological index data will hereinafter be referred to collectively as "biological data".

As shown in FIG 1, the apparatus 1 has a main body 2 comprising a base 21 and a load-receiving platform 22 mounted on the base 21. The platform 22 has a top surface 22a provided with a pair of current supply electrodes 31, 32 for supplying an AC current between the right and left feet bottoms of a user, a pair of measurement electrodes 41, 42 for measuring a voltage (potential difference) arising between the right and left feet in response to the current supply, a liquid-crystal display screen 5 for displaying personal data and biological data, and an input device 6 for allowing the user to enter personal data therethrough.

The input device 6 includes an up key 61, a down key 62 and a set key 63 which are used in an operation for selecting and setting a datum or data to be displayed on the liquid-crystal display screen 5. The main body 2 has a side surface provided with a set of foot keys 7 consisting of four foot keys 71, 72, 73, 74 which are used for calling up registered personal data if any, and a foot key 8 for turning off power. Each of the set key 63 and the foot keys 71 to 74 doubles as a power-on key. Further, as indicated by the dashed circle in FIG 1, the top surface 22a of the platform 22 is provided with a set of light emitters 9 consisting of after-mentioned nine LEDs (light-emitting diodes) 91, 92, 93, 94, 95, 96, 97, 98, 99.

As shown in FIG 2, the apparatus 1 is internally provided with a current supply circuit 11 electrically connected to the current supply electrodes 31, 32, a voltage measurement circuit 12 electrically connected to the measurement electrodes 41, 42, a weight sensor 13 adapted to generate an output corresponding to a load imposed thereon so as to measure body weight data of the user, an A/D converter 14 for converting a voltage signal from the voltage measurement circuit 12 and the weight sensor 13 to a digital signal, an input/output circuit 15 electrically connected to each of the liquid-crystal display screen 5, the input device 6, the foot keys 7, the power-off key 8 and the light emitters 9 (LEDs 91 to 99), a storage device 16 for storing the entered personal data and the measured biological data, a power supply device 17 including a battery, and a control device 10 electrically connected to each of the current supply circuit 11, the A/D converter 14, the input/output circuit 15, the storage device 16 and the power supply device 17.

The above control device 10 is provided with a conventional computing element (CPU), and adapted to continuously update current date/hour according to a built-in clock and execute a control program pre-stored on the storage device 16 so as to measure biological data of a user by performing various control processes, such as a process of receiving personal data entered from the input device 6; a process of measuring body weight data using the weight sensor 13; a process of supplying an AC current to the current supply electrodes 31, 32; a process of calculating a bioelectric impedance of the user in accordance with the supplied current value and a voltage value detected by the measurement electrodes 41, 42; a process of calculating body composition data and biological index data in accordance with the calculated bioelectric impedance, the personal data entered from the input device 4 and the body weight data measured by the weight sensor 13; a process of displaying the entered personal data and the measured biological data on the liquid-crystal display screen 5; and a process of storing these data on the storage device 16.

Particularly, in the apparatus 1 according to this embodiment, the light emitters 9 (LEDs 91 to 99) correspond, respectively, to nine kinds of biological data to be measured by this apparatus 1, and the control device 10 is operable to perform a control process of displaying each of the biological data on the liquid-crystal display screen 5, one-by-one automatically or according to each selection operation performed by the user, and lighting or activating the light emitter 9 corresponding to the displayed biological datum. With reference to FIGS. 3 to 7(D), this control process will be described below.

The flowchart of FIG 3 shows a process of registering personal data, for example, when a user uses the apparatus 1 for the first time. When a user presses down the set key 63 to activate the apparatus 1, this registration process is performed.

In Step S1, a personal code of the user is set up. Specifically, four numerals 1 to 4 are firstly displayed as alternative personal codes on the liquid-crystal display screen 5, and the user selects any one of the displayed numerals using the up key 61 and/or the down key 62, and sets up the selected numeral as his/her personal code using the set key 63. Then, in Step S2, it is determined whether there are any previously registered personal data corresponding to the selected personal code. When the determination in Step S2 is YES or there are the reregistered personal data, the process advances to Step S3. If the determination in Step S2 is NO or there is no registered personal data, the process will advance to Step S4. In Step S3, a selection message requesting to determine whether a re-registration is performed is displayed on the liquid-crystal display screen 5. If the user selects the re-registration, the process will advance to Step S4. When the user selects the non-re-registration, the registration process is terminated and the power supply is automatically turned off.

In Step S4, the birth date of the user is entered. Specifically, numerals representing year, month and day are displayed on the liquid-crystal display screen 5, and the user increases/reduces each of the numerals using the up key 61 and/or the down key 62 to adjust the numerals to his/her own birth date, and sets up the adjusted numerals as his/her birth date using the set key 63. Then, the control device 10 compares the entered birth date with a current date indicated by the built-in clock to calculate an age datum. Then, in Step S5, a sexuality datum of the user is entered. Specifically, a selection message requesting to select either one of man and woman is displayed on the liquid-crystal display screen 5, and the user selects his/her sexuality using the up key 61 and/or the down key 62, and sets up the selected sexuality using the set key 63. Then, in Step S6, a body height datum of the user is entered. Specifically, numerals representing body height are displayed on the liquid-crystal display screen 5, and the user increases/reduces each of the numerals using the up key 61 and/or the down key 62 to adjust the numerals to his/her own body height, and sets up the adjusted numerals as his/her body height using the set key 63.

In Step S7, the personal code selected in Step S 1 and the personal data entered in Steps S4 to S6 are associated with each other, and stored on the storage device 16. Through the above steps, the personal-data registration process is completed, and, after a given time period (e.g. about 10 seconds) has elapsed from the completion, the power supply will be automatically turned off. During this given time period, the entered personal data may be displayed on the liquid-crystal display screen 5 to allow the user to confirm them. It is to be understood that the user may press the power-off key 8 to turn off power.

The flowchart of FIG 4 shows a process of measuring biological data, such as user's own body composition or biological index, using the apparatus 1. When the user presses down one of the foot keys 7 (71 to 74) which corresponds to the personal code associated with the user's own personal data to activate the apparatus 1, this measurement process is performed.

In Step S11, it is determined whether there are any previously registered personal data corresponding to the pressed foot key 7. If the determination in Step S11 is YES or there are the reregistered personal data, the process will advance to Step S12 to read the registered personal data and then advance to Step S13. When the determination in Step S11 is NO or there is no registered personal data, the measurement process is terminated. Before the termination, a message indicative of non-registration of personal data may be displayed on the liquid-crystal display screen 5 for a given time period to prompt the user to register his/her personal data, or the process may be shifted to Step S4 in FIG 3.

In Step S13, it is determined whether the set key 63 has been pressed down. If the determination in Step 13 is YES or the set key 63 has been already pressed down at this moment, the process will advance to Step S17 in which the control device 10 will perform the after-mentioned process of displaying past biological data stored in association with the personal code. When the determination in Step S 13 is NO or the weight sensor detects a load without pressing of the set key 63, the process advances to Step S 14 to measure biological data.

In Step S 14, data about a body weight of the user and a bioelectric impedance between the feet of the user are measured. Specifically, when the user stands on the top surface 22a of the platform 22 in the posture where the left foot bottom is in contact with the current supply electrode 31 and the measurement electrode 41 and the right foot bottom is in contact with the current supply electrode 32 and the measurement electrode 42, the body weight datum of the user is measured based on a load detected by the weight sensor 13. Simultaneously, an AC current is supplied between the right/left foot bottoms through the current electrodes 31, 32, and a voltage (potential difference) between the right/left foot bottoms is measured through the measurement electrodes 41, 42. Then, an inter-foot bioelectric impedance is calculated based on the supplied current value and the measured voltage. Alternatively, a plurality of reference resistors each having a different known resistance value may be provided in the electric circuit in series or parallel to the user's body, to obtain a potential differences caused by each of the resistors together with a potential difference arising between the user's feet, and calculate a user's bioelectric impedance in accordance with the ratio between each of the obtained potential differences and each resistance value of the reference resistors. In this case, the impedance datum can be obtained even if the current value to be supplied to the user's body is unknown.

Then, in Step S15, body composition data, such as body fat percentage, body water percentage, muscle mass, basal metabolic rate and/or bone mass, and biological index data, such as visceral fat level, physique level and/or metabolic age, are calculated based on the registered age, sexuality and body height data serving as the personal data of the user, and the measured body weight and bioelectric impedance data obtained in Step S 14. Then, the biological data consisting of the body composition and biological index data, and the body weight datum measured in Step S14, are stored on the storage device 16 in association with the personal code of the user. Each of the above body composition and biological index data is calculated according to individual calculation formulas pre-stored on the storage device 16. Most of the calculation formulas are prepared by using all or a part of the above personal data, weight datum and impedance datum, as parameter. Particularly, in the calculation formulas for body fat percentage and body water percentage as fundamental biological data, at least data about body height, body weight and bioelectric impedance, are incorporated therein as parameters. The step of measuring biological data using such calculation formulas is the same as that in the conventional body composition analyzer, and it is not related directly to the feature of the present invention. Thus, its further detailed description will be omitted.

Then, in Step S16, each of the latest biological data measured at Steps S14 and S15 is displayed on the liquid-crystal display screen 5 while lighting or activating the corresponding light emitter 9. The control in Step S16 will be described below with reference to the flowchart of FIG 5.

In Step S161, a timer for controlling a display time-period of each biological datum is firstly reset. This timer determines an elapsed time from the initiation of this latest-biological-data display process. The built-in clock of the control device 10 is used as the timer.

Then, in Step S162, the display of each of the biological data onto the liquid-crystal display screen 5 and the lighting or activation of the corresponding light emitter 9 are performed according to a first designation number X programmed in such a manner that it is initially set to "1" and incremented one-by-one in the after-mentioned Step S170 (wherein the value of the first designation number X is returned to "1" after it reaches "9"), and a second designation number Y programmed in such a manner that it is initially set to "1" and incremented by one in the after-mentioned Step S169.

The first designation number X is a parameter for determining the biological datum to be displayed and the light emitter 9 to be activated. More specifically, designation numbers "1" to "9" are pre-assigned, respectively, to the nine kinds of biological data to be measure by the apparatus 1 and the corresponding nine LEDs 91 to 99, and the control device 10 is operable, in response to a current value of the first designation number X, to select one of the biological data assigned with the current value so as to display it on the liquid-crystal display screen 5, and simultaneously select one of the light emitters 9 assigned with the current value so as to activate it.

The second designation number Y is a parameter for determining a lighting mode of the light emitters 9 during the display of the latest biological data. Specifically, the lighting pattern or mode of the light emitters 9 during the display of the latest biological data includes two kinds: "normal lighting" and "medium-speed blinking", which are assigned, respectively, with designation numbers "1" and "2". The control device 10 is operable, in response to a current value of the second designation number Y, to activate the light emitter 9 in the lighting mode assigned with the current value.

Then, in Step S163, it is determined whether the up key 61 or the down key 62 has been pressed down. If the determination in Step S163 is NO or none of the up key 61 and the down key 62 has been pressed down, the process will advance to Step S 164 to display the biological data and activate the corresponding light emitters 9 according to the automatic data selection at given time intervals (automatic/periodical data selection). When the determination in Step S163 is YES or either one of the up key 61 and the down key 62 has been pressed down, the process advances to Step S 167 to display the biological data and activate the corresponding light emitters 9 according to the user's selection operation.

In Step S164, it is determined whether the second designation number Y about the lighting mode has a value of "2". If the determination in Step S164 is YES or the second designation number Y = "2", the process will skip Step S165 and advance to Step S166. When the determination in Step S 164 is NO or the second designation number Y ≠ "2" (or Y = "1"), the process advances to Step S165.

The second designation number Y is initially set to "1" and incremented by one in the after-mentioned Step S169 to which the process advances when the determination in Step S163 is YES or either one of the up key 61 and the down key 62 is pressed down. Thus, the second designation number Y is kept at "1" (Y ≠ "2") unless the user presses down either one of the up key 61 and the down key 62 once, and thereby the process will advance to Step S165. In contrast, when the user presses down either one of the up key 61 and the down key 62 once, the second designation number Y is changed to "2" in Step S169 as described later. Subsequently, the process will always skip Step S165 and advance to Step S166.

In Step S165, it is determined whether 3 × x seconds have elapsed from the timer reset in Step S161. When the determination in Step S165 is YES or 3 × x seconds have elapsed, the process advances to the after-mentioned Step S170 to increment the first designation number X by one and then advance the process to Step S166. If the determination in Step S165 is NO or 3 × x seconds have not elapsed, the process will advance to Step S166 without going through Step S 170. In Step S166, it is determined whether 30 seconds have elapsed from the timer reset in Step S161 or the after-mentioned Step S167. If the determination in Step S166 is YES or 30 seconds have elapsed, the biological-data display process will be fully completed. When the determination in Step S166 is NO or 30 seconds have not elapsed, the process returns to Step S162.

Fundamentally, the same value as that of the first designation number X is assigned to the value x of the 3 × x seconds in Step S165. For example, when the first designation number X is "1", the value x is "1", and it is determined whether 3 (3 × 1) seconds have elapsed from the timer reset in Step S161. When the first designation number X is "5", the value x is "5", and it is determined whether fifteen (3 × 5) seconds have elapsed from the timer reset in Step S161. While the value of the first designation number X is programmed in such a manner as to be returned to "1" after it reaches "9", the value x is programmed in such a manner as to be stepwise increased to 10, 11, 12, ---- even after it reaches "9".

Through Steps S161 to S166 and Step S 170, the biological-data display and the lighting of the corresponding light emitters 9 are performed based on the automatic/periodical data selection. Specifically, in Step S162, the biological datum with the first designation number X of "1" is firstly displayed. Then, when 3 seconds have elapsed without pressing of the up key 61 and the down key 62, the value of the first designation number X is set to "2" in Step S170, and the biological datum with the first designation number X of "2" is switchingly displayed. Subsequently, the biological datum with the first designation number X of "3" is switchingly displayed when 3 seconds have further elapsed (after 6 (3 × 2) seconds from the timer reset), and the biological datum with the first designation number X of "4" is switchingly displayed when 3 seconds have further elapsed (after 9 (3 × 3) seconds from the timer reset). In this manner, each of the nine kinds of biological data will be automatically selected one-by-one at 3-second intervals and displayed on the liquid-crystal display screen 5. In conjunction with this display, the light emitter 9 assigned with the same first designation number X as that of the selected biological datum is activated (lighted) in the lighting mode defined by the second designation number Y of "1". These display and lighting controls will continue for total 30 seconds from the timer reset in Step S161 or the initiation of the latest-biological-data display process. It is to be understood that the above time values, such as 3 seconds and 30 seconds, may be appropriately set in consideration of user-friendliness.

When the determination in Step S163 is YES or either one of the up key 61 and the down key 62 has been pressed down, the process advances to Step S167. In Step S167, the timer initially reset in Step S161 is reset again. Thus, the timer determines an elapsed time from the pressing of the up key 61 or the down key 62.

Then, in Step S168, it is determined whether the second designation number Y about the lighting mode has a value of "2". When the determination in Step S168 is YES or the value of the second designation number Y is "2", the process skips Step S169 and advances to Step S 170. If the determination in Step S168 is NO or the value of the second designation number Y is not "2" (Y= "1"), the process will advance to Step S169. As described above, the initial value of the second designation number Y is set at "1". Thus, if the process advances to Step S168 after either one of the up key 61 or the down key 62 has been pressed down for the first time, the process will obviously advance to Step S169.

In Step S169, the value of the second designation number Y is incremented by one or becomes "2". Thus, the light emitter 9 is activated in the lighting mode defined by the second designation number Y of "2" (or blinked on and off at a medium speed). After the second designation number Y is changed to "2", Step S169 will be skipped according to the determination in Step S168.

In Step S170, the first designation number X is incremented by one. Specifically, when the first designation number X has a current value of "1", the value is changed to "2". When the first designation number X has a current value of "2", the value is changed to "3". As described above, the first designation number X is programmed in such a manner as to be retuned to "1" when it reaches "9". After completion of Step S170, the process advances to Step S166. In Step S166, it is determined whether 30 seconds have elapsed from the timer reset in Step S167, or whether 30 seconds have elapsed from the pressing of the up key 61 or the down key 62.

In the process flow between Step S163 and Step S166 through Step S167, the display control of the biological data and the lighting control of the light emitters 9 according to the user's selection operation are performed. That is, one of the designation numbers X (or one of the nine kinds of biological data) is selected according to the user's selection operation or the operation of pressing down the up key 61 or the down key 62, and the biological datum freely selected by the user will be displayed on the liquid-crystal display screen 5 until the user performs a next selection operation. Thus, the biological datum to be displayed on the liquid-crystal display screen 5 is switched every time the user presses down the up key 61 or the down key 62. Simultaneously, the light emitter 9 assigned with the same first designation number X as that of the selected biological datum is activated in the lighting mode defined by the second designation number of "2" (or blinked at a medium speed). These display and lighting controls will continue for total 30 seconds from the timer reset in Step S 167 or the time when the user lastly presses down the up key 61 or the down key 62. For example, the program for switching the biological data may be configured to increment the first designation number X one-by-one when the up key 61 is pressed down, and decrement the first designation number X one-by-one when the down key 62 is pressed down. This program makes it possible to reverse the order of biological data switching depending on the pressed keys.

When the determination in Step S166 is YES or 30 seconds have elapsed, the latest-biological-data display process is completed and thereby the entire biological-data measurement process is completed.

Returning to the flowchart of FIG 4, a process to be performed when the determination in Step S13 is YES or the set key 63 has been pressed, will be described. In this process, the set key 63 is pressed to display past biological data stored in the storage device 16. When the determination in Step S 13 is YES or the set key 63 has been pressed, the process advances to Step S 17.

In Step S 17, a measurement date of biological data to be displayed is entered. Specifically, numerals representing year, month and day are displayed on the liquid-crystal display screen 5, and the user increases/reduces each of the numerals using the up key 61 and/or the down key 62 to adjust the numerals to the past measurement date, and sets up the adjusted numerals as the measurement date using the set key 63. It is to be understood that the apparatus 1 may be designed to enter the member of elapsed days from this measurement or the number of times of measurements before this measurement, in place of the measurement date.

Then, in Step S 18, the biological data having the measurement date entered in Step S 17 is read out. If no corresponding data is stored, the process may be terminated after displaying an error message indicative of such information, or may return to Step S 17.

Then, in Step S19, the past biological data read in Step S18 are displayed on the liquid-crystal display screen 5, and simultaneously the corresponding light emitters 9 are activated. A control in Step S 19 will be described below with reference to the flowchart of FIG 6. The flowchart of FIG 6 is fundamentally similar to that of FIG. 5, and its description other than a different point will be simply described.

In Step S 191, the timer is reset in the same manner as that in Step S161.

Then, in Step S192, the display control of each of the biological data and the lighting control of the corresponding light emitter 9 are performed according to the first designation number X in the same manner as that in Step S162, except that the biological data to be displayed are the past biological data read in Step S18, and the lighting mode (lighting pattern) of the light emitters 9 is determined by a third designation number Z instead of the second designation number Y. The third designation number Z is programmed in such a manner as to be initially set to "1" and incremented by one in the after-mentioned Step S199, wherein the light emitters are blinked at a low speed (activated in a low-speed blinking mode) when the third designation number Z is "1", and blinked at a high speed (activated in a high-speed blinking mode) when the third designation number Z is "2". In this manner, the lighting mode is prepared differently between the latest-biological-data display and the past-biological-data display, and the designation number is set differently between the respective lighting modes. Thus, the lighting mode of the light emitters 9 during the latest-biological-data display can be changed from that during the past-biological-data display.

Then, in Step S 193, it is determined whether the up key 61 or the down key 62 has been pressed down, in the same manner as that in Step S163. If the determination in Step S 193 is NO or none of the up key 61 and the down key 62 has been pressed down, the process will advance to Step S194. When the determination in Step S193 is YES or either one of the up key 61 and the down key 62 has been pressed down, the process advances to Step S 197.

In Step S194, it is determined whether the third designation number Z has a value of "2". When the determination in Step S194 is NO or the third designation number Y ≠ "2", the process advances to Step S195. If the determination in Step S194 is YES or the third designation number Y = "2", the process will skip Step S 195 and advance to Step S 196.

In Step S195, it is determined whether 3 × x seconds have elapsed from the timer reset in Step S191, in the same manner as that in Step S165. When the determination in Step S 195 is YES or 3 × x seconds have elapsed, the process advances to Step S 196 through Step S200. If the determination in Step S195 is NO or 3 × x seconds have not elapsed, the process will advance to Step S 196 without going through Step S200.

In Step S 196, it is determined whether 30 seconds have elapsed from the timer reset in Step S191 or the after-mentioned Step S197, in the same manner as that in Step S166. If the determination in Step S196 is YES or 30 seconds have elapsed, the past-biological-data display process will be completed. When the determination in Step S196 is NO or 30 seconds have not elapsed, the process returns to Step S192.

In Step S197, the timer is reset in the same manner as that in Step S167, and then the process advances to Step S 198.

Then, in Step S 198, it is determined whether the third designation number Z has a value of "2". If the determination in Step S 198 is NO or the value of the second designation number Y ≠ "2", the process will advance to Step S199. When the determination in Step S 198 is YES or the value of the second designation number Y = "2", the process skips Step S199 and advances to Step S200.

In Step S 199, the value of the third designation number Z is incremented by one or becomes "2". Thus, the light emitters 9 are activated in the lighting mode defined by the third designation number "2" (blinked at a high speed).

In Step S200, the first designation number X is incremented by one, in the same manner as that in Step S 170, and then the process advances to Step S 196. When the determination in Step S196 is YES or 30 seconds have elapsed, the past-biological-data display process is completed and thereby the entire biological-data measurement process is completed.

FIG 7(A) to 7(D) illustrate the display of the liquid-crystal display screen 5 and the lighting mode of the light emitters 9 in the apparatus 1. The light emitters 9 or the nine LEDs 91 to 99 are disposed adjacent to the liquid-crystal display screen 5. The LEDs 91 to 99 are covered, respectively, with nine light-transparent films 91a, 92a, 93a, 94a, 95a, 96a, 97a, 98a, 99a. Each of the films 91a to 99a has a mark representing the biological datum associated with the LED covered therewith.

FIG. 7(A) shows one example of a display about body weight (first designation number X = "1") as one of the biological data. A value "73.1" indicative of body weight and its unit "kg" are displayed on the liquid-crystal display screen 5, and the LED 91 covered with the film 91a having a mark representing body weight (weight mark) is lighted or activated in a normal lighting mode (second designation number Y = 1). That is, this screen currently displays a body weight datum which is a latest datum just after being measured, and will be automatically switched to display another biological datum (first designation number X = "2") after 3 seconds.

FIG 7(B) shows one example of a display about body fat percentage (first designation number X = "2") as another of the biological data. A value "21.7" indicative of body fat percentage and its unit "%" are displayed on the liquid-crystal display screen 5, and the LED 92 covered with the film 92a having a mark representing body fat percentage (mark having a white symbol "%" on a human-body-shaped colored background) is activated in the medium-speed blinking mode (second designation number Y = 2). That is, this screen currently displays a body fat percentage datum which is a latest datum just after being measured, and will be continuously displayed for 30 seconds unless the user presses either one of the up key 61 or the down key 62.

FIG 7(C) shows one example of a display about bone mass (first designation number X = "8") as still another of the biological data. A value "4.7" indicative of bone mass and its unit "kg" are displayed on the liquid-crystal display screen 5, and the LED 98 covered with the film 98a having a mark representing bone mass (mark having while bone-shaped lines on a human-body-shaped colored background) is activated in the low-speed blinking mode (third designation number Z = "1"). That is, this screen currently displays a bone mass datum which is stored in the storage device 16 after being measured at a certain past time, and will be automatically switched to display another biological datum (first designation number X = "9") after at least 3 seconds.

FIG. 7(D) shows one example of a display about visceral fat level (first designation number X = "9") as yet another of the biological data. A value "9" indicative of visceral fat level is displayed on the liquid-crystal display screen 5, and the LED 99 covered with the film 99a having a mark representing visceral fat level (mark having a white abdominal section on a human-body-shaped colored background) is activated in the high-speed blinking mode (third designation number Z = 2). That is, this screen currently displays a visceral fat level datum which is stored in the storage device 16 after being measured at a certain past time, and will be continuously displayed for 30 seconds unless the user presses either one of the up key 61 or the down key 62.

As described above, in the apparatus 1 according to the first embodiment, data measurement means primarily composed of the input device 6, the weight sensor 13, the current-supply electrodes 31, 32, the current-supply circuit 11, the measurement electrodes 41, 42, the voltage measurement circuit 12, and the control circuit 10, measures the plural (nine) kinds of biological data about a user, as in Steps S4 to S6 and Steps S14 and S15. Then, data selection means primarily composed of the input device 6 and the control device 10 selects one of the measured biological data as in Steps S163 to S170 or Steps S193 to S200, and data display means primarily composed of the liquid-crystal display screen 5 and the control device 10 displays the selected biological datum as in Step S162 or S 192. In particular, the plurality (nine) of light emitters 9 primarily composed of the LEDs 91 to 99 are provided correspondingly to the biological data, and lighting control means primarily composed of the control device 10 activates the light emitter 9 corresponding to the selected biological datum, as in Step S162 or S 192.

The LEDs 91 to 99 constituting the light emitters 9 are covered, respectively, with the light-transparent films 91a to 99a each having a mark representing the corresponding biological datum.

Further, the data selection means primarily composed of the input device 6 and the control device 10 includes automatic data-selection means adapted to automatically select each of the plural kinds of biological data one-by-one at given time (3-second) intervals, as in Steps S165 and S 170 or Steps S 195 and S200, and arbitrary data-selection means adapted to arbitrarily select one of the plural kinds of biological data according to a selection operation (pressing of the up key 61 or the down key 62) performed by the user, as in Steps S163 and S 170 or Steps S 193 and S200. The lighting control means primarily composed of the control device 10 activates the light emitters 9 in a first lighting mode (normal lighting mode or low-speed blinking mode) when the selection of the biological data is performed by the automatic data-selection means, and in a second lighting mode (medium-speed blinking mode or high-speed blinking mode) when the selection of the biological data is performed by the arbitrary data-selection means, as in Steps S162 and S169 or Steps S 192 and S 199.

Furthermore, data storage means primarily composed of the storage device 16 stores the plural kinds of biological data measured, as in Step 15, and the data selection means primarily composed of the input device 6 and the control device 10 includes latest-data selection means adapted to select one of latest biological data newly measured by the data measurement means, as in Steps S13 and S16, and past-data selection means adapted to select one of past biological data stored on the data storage means, as in Steps S13 and S19. The lighting control means activates the light emitters 9 in a third lighting mode (normal lighting mode and medium-speed blinking mode) when the selection of the biological data is performed by the latest-data selection means, and in a fourth lighting mode (low-speed blinking mode or high-speed blinking mode) when the selection of the biological data is performed by the past-data selection means, as in Steps S162 and S192.

### [SECOND EMBODIMENT]

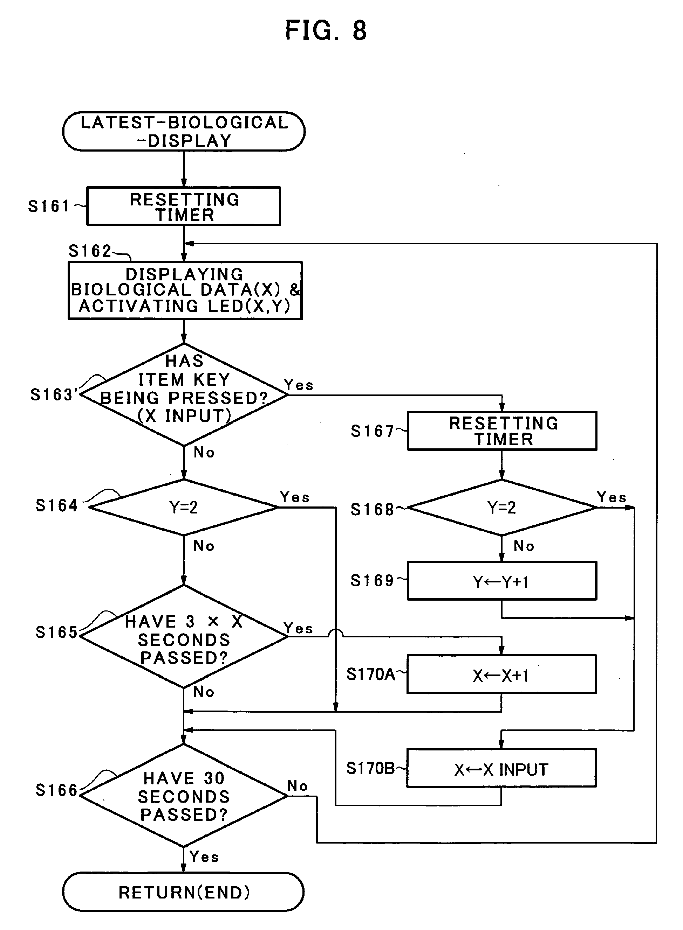

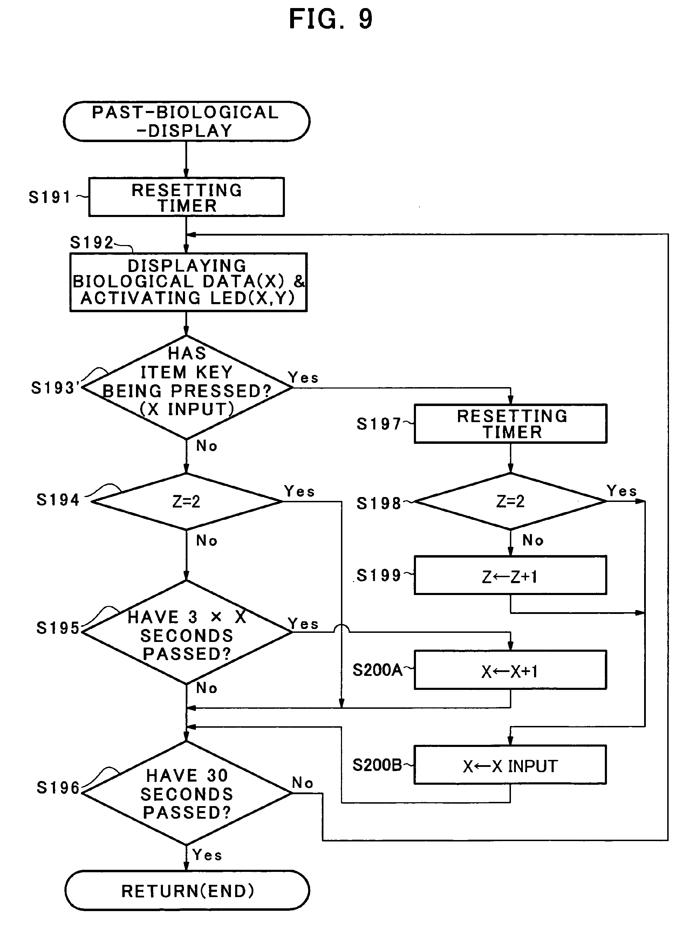

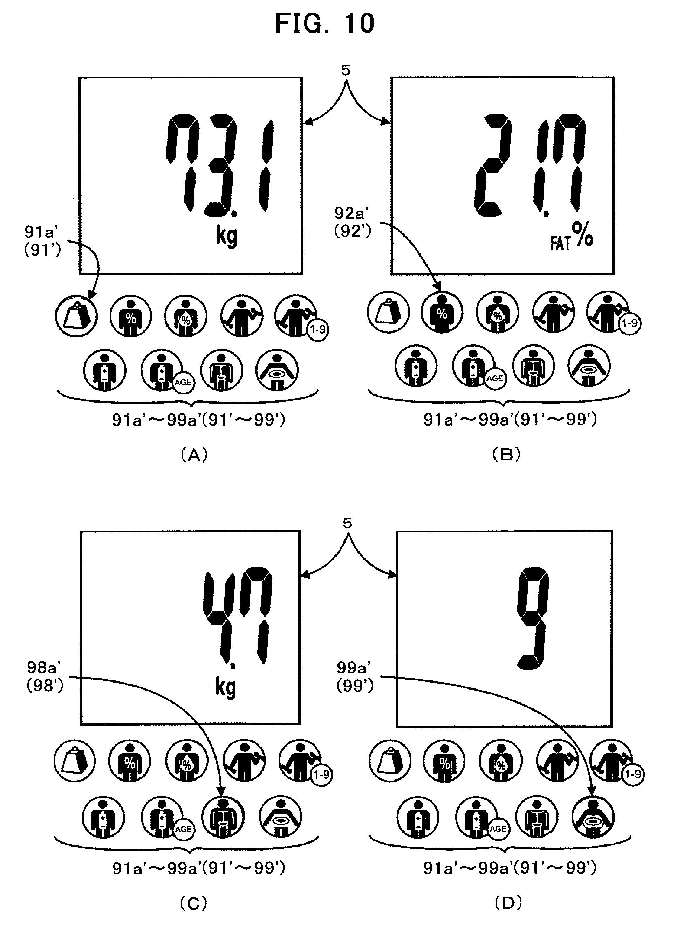

With reference to the drawings, a second preferred embodiment of the present invention will be described below. FIGS. 1 to 4 used in the description about the first embodiment are also used in the following description about the second embodiment. FIG. 8 is a flowchart of a latest-biological-data display process to be performed in a biological data measurement apparatus 1' according to the second embodiment, and FIG. 9 is a flowchart of a latest-biological-data display process to be performed in the biological data measurement apparatus 1'. FIGS. 10(A) to 10(D) are explanatory diagrams of various examples of a display of a liquid-crystal display screen and a lighting mode of light emitters in the biological data measurement apparatus 1'.

This biological data measurement apparatus 1' (hereinafter referred to simply as "apparatus 1' ") has no major difference from the biological data measurement apparatus 1 according to the first embodiment, except that a plurality of item keys 9' each having a light emitter incorporated therein is used in place of the light emitters 9 in the first embodiment, and the latest-biological-data display process and the latest-biological-data display process in the first embodiment are partly modified in connection with the use of the item keys 9'. Thus, an element or component defined by the same reference numeral is identical to that in the first embodiment unless otherwise specified.

As indicated by the dashed circle in FIG. 1, the top surface 22a of the platform 22 in the apparatus 1' is provided with nine item keys 9' between the liquid-crystal display screen 5 and the input device 6. The item keys 9' consist of nine key switches 91', 92', 93', 94', 95', 96', 97', 98', 99' each incorporating a LED (light-emitting diode) serving as a light emitter. The item keys 9' (key switches 91' to 99') are associated, respectively, with nine kinds of biological data to be measured by the apparatus 1'. The apparatus 1' is operable, when a user presses down one of the item keys to perform a selection operation, to display one of the biological data which is associated with the selected item key, and simultaneously activate one of the light emitters which corresponds to the displayed biological datum (or which is incorporated in the selected item key) so as to light the selected item key 9' itself. This control process will be described, particularly, with reference to FIGS. 8 to 10.

When a biological-data measurement process (FIG 4) advances to Step S16, a latest-biological-data display process illustrated in the flowchart of FIG 8 is executed.

In Step S161, a timer for controlling a display time-period of each biological datum is reset. This timer determines an elapsed time from the initiation of this latest-biological-data display process. The control device 10 has a built-in clock used as the timer.

Then, in Step S162, the display of each of the biological data onto the liquid-crystal display screen 5 and the lighting or activation of the light emitter incorporated in the item key 9' associated with the displayed biological datum are performed according to a first designation number X programmed in such a manner that it is initially set to "1" and incremented one-by-one in the after-mentioned Step S170A (wherein the value of the first designation number X is returned to "1" after it reaches "9") or it has a value corresponding to each of the item keys 9' (key switches 91' to 99') in the after-mentioned Step S170B, and a second designation number Y programmed in such a manner that it is initially set to "1" and incremented by one in the after-mentioned Step S169.

The first designation number X is a parameter for determining the biological datum to be displayed and the item key 9' incorporating the light emitter to be activated. More specifically, designation numbers "1" to "9" are pre-assigned, respectively, to the nine kinds of biological data to be measure by the apparatus 1' and the corresponding nine key switches 91' to 99' of the apparatus 1', and the control device 10 is operable, in response to a current value of the first designation number X, to select one of the biological data assigned with the current value so as to display it on the liquid-crystal display screen 5, and simultaneously select one of the light emitters (item keys 9') assigned with the current value so as to activate it.

The second designation number Y is a parameter for determining a lighting mode of the item keys 9' each incorporating the light emitter. Specifically, in this apparatus 1', the lighting mode of the light emitters (item keys 9') during the display of the latest biological data includes two kinds: "normal lighting" and "medium-speed blinking", which are assigned, respectively, with designation numbers "1" and "2". The control device 10 is operable, in response to a current value of the second designation number Y, to light the item key 9' incorporating the light emitter in the lighting mode assigned with the current value.

Then, in Step S163', it is determined whether one of the item key 9' has been pressed down and, if YES, the designation number of the pushed item key 9' (this designation number will be expressed by "Xinput" for convenience sake) is checked. If the determination in Step S163 is NO or none of the item key 9' has been pressed down, the process will advance to Step S 164 to display the biological data and light the corresponding item keys 9' according to a selection operation performed automatically at given time intervals (automatic/periodical data selection). When the determination in Step S163 is YES or either one of the item key 9' has been pressed down, the process advances to the after-mentioned Step S167 to display the biological data and light the corresponding item keys 9' each incorporating the light emitter, according to a selection operation performed by a user.

In Step S164, it is determined whether the second designation number Y about the lighting mode has a value of "2". If the determination in Step S164 is YES or the second designation number Y = "2", the process will skip Step S165 and advance to Step S166. When the determination in Step S164 is NO or the second designation number Y ≠ "2" (or Y = "1"), the process advances to Step S165.

The second designation number Y is initially set to "1" and incremented by one in the after-mentioned Step S 169 to which the process advances when the determination in Step S163' is YES or either one of the item keys 9' is pressed down. Thus, the second designation number Y is kept at "1" (Y ≠ "2") unless the user presses down either one of the item keys 9' once, and thereby the process will advance to Step S165. In contrast, when the user presses down either one of the item keys 9' once, the second designation number Y is changed to "2" in Step S 169 as described later. Subsequently, the process will always skip Step S165 and advance to Step S166.

In Step S165, it is determined whether 3 × x seconds have elapsed from the timer reset in Step S161. When the determination in Step S165 is YES or 3 × x seconds have elapsed, the process advances to Step S166 through Step S170A. If the determination in Step S165 is NO or 3 × x seconds have not elapsed, the process will advance to Step S166 without going through Step S170A. Fundamentally, the same value as that of the first designation number X is assigned to the value x of the 3 × x seconds in Step S165. For example, when the first designation number X is "1", the value x is "1", and it is determined whether 3 (3 x 1) seconds have elapsed from the timer reset in Step S161. When the first designation number X is "5", the value x is "5", and it is determined whether fifteen (3 × 5) seconds have elapsed from the timer reset in Step S161. While the value of the first designation number X is programmed in such a manner as to be returned to "1" after it reaches "9", the value x is programmed in such a manner as to be stepwise increased to 10, 11, 12, ---- even after it reaches "9".

In Step S170A, the value of the first designation number X is incremented by one. For example, when a current value of the first designation number X is "1", it will be changed to "2". When a current value of the first designation number X is "2", it will be changed to "3". Further, as mentioned above, the first designation number X is programmed in such a manner as to be returned to "1" after it reaches "9".

After it is determined in Step S164 that Y is "2" or it is determined in Step S165 that 3 × x seconds have not elapsed or the value of the first designation number X is incremented by one in Step S170A, the process advances to Step S166. In Step S166, it is determined whether 30 seconds have elapsed from the timer reset in Step 161 or the after-mentioned Step S167. When the determination in Step S166 is YES or 30 seconds have elapsed, this biological-data display process is completed. If the determination in Step S166 is NO or 30 seconds have not elapsed, the process will return to Step S162.

Through Steps S161 to S166 and Step S170A, the biological-data display and the lighting of the corresponding item keys 9' each incorporating the light emitter are performed based on the automatic/periodical data selection. Specifically, in Step S162, the biological datum with the first designation number X of "1" is firstly displayed. Then, when 3 seconds have elapsed without pressing of the item keys 9', the value of the first designation number X is set to "2" in Step S170A, and the biological datum with the first designation number X of "2" is switchingly displayed. Subsequently, the biological datum with the first designation number X of "3" is switchingly displayed when 3 seconds have further elapsed (after 6 (3 × 2) seconds from the timer reset), and the biological datum with the first designation number X of "4" is switchingly displayed when 3 seconds have further elapsed (after 9 (3 × 3) seconds from the timer reset). In this manner, each of the nine kinds of biological data will be automatically selected one-by-one at 3-second intervals and displayed on the liquid-crystal display screen 5. In conjunction with this display, the item key 9' incorporating the light emitter assigned with the same first designation number X as that of the selected biological datum is activated (lighted) in the lighting mode defined by the second designation number Y of "1". These display and lighting controls will continue for total 30 seconds from the timer reset in Step S161 or the initiation of the latest-biological-data display process. It is to be understood that the above time values, such as 3 seconds and 30 seconds, may be appropriately set in consideration of user-friendliness.

When the determination in Step S163' is YES or either one of the item keys 9' has been pressed down, the process advances to Step S167. In Step S167, the timer initially reset in Step S161 is reset again. Thus, the timer determines an elapsed time from the pressing of the item key 9'.

Then, in Step S168, it is determined whether the second designation number Y about the lighting mode has a value of "2". When the determination in Step S168 is YES or the value of the second designation number Y is "2", the process skips Step S169 and advances to Step S170B. If the determination in Step S168 is NO or the value of the second designation number Y is not "2" (Y = "1"), the process will advance to Step S169. As described above, the initial value of the second designation number Y is set at "1". Thus, if the process advances to Step S168 after either one of the item keys 9' has been pressed down for the first time, the process will obviously advance to Step S169.

In Step S169, the value of the second designation number Y is incremented by one or becomes "2". Thus, the item key 9' incorporating the light emitter is activated in the lighting mode defined by the second designation number Y of "2" (or blinked on and off at a medium speed). After the second designation number Y is changed to "2", Step S169 will be skipped according to the determination in Step S168.

In Step S170B, the first designation number X is changed depending on the designation number Xinput (either one of "1" to "9") assigned to the item key 9' pressed down in Step S163'. Specifically, when the designation number of the pressed item key 9' is "2", the first designation number X is changed to "2". When the designation number of the pressed item key 9' is "3", the first designation number X is changed to "3". After completion of Step S170B, the process advances to Step S166. In Step S166, it is determined whether 30 seconds have elapsed from the timer reset in Step S167, or whether 30 seconds have elapsed from the pressing of the item key 9'.

In the process flow between Step S163' and Step S166 through Step S167, the display control of the biological data and the lighting control of the item keys 9' each incorporating the light emitter, according to the user's selection operation using the item keys 9' (key switches 91' to 99') are performed. That is, one of the designation numbers X (or one of the nine kinds of biological data) is selected according to the user's selection operation or the operation of pressing down one of the item keys 9', and the biological datum freely selected by the user will be displayed on the liquid-crystal display screen 5 until the user performs a next selection operation. Thus, the biological datum to be displayed on the liquid-crystal display screen 5 is switched every time the user presses down one of the item keys 9'. Simultaneously, the item key 9' incorporating the light emitter assigned with the same first designation number X as that of the selected biological datum is lighted in the lighting mode defined by the second designation number of "2" (or blinked at a medium speed). These display and lighting controls will continue for total 30 seconds from the timer reset in Step S167 or the time when the user lastly presses down the item key 9'.

When the determination in Step S166 is YES or 30 seconds have elapsed, the latest-biological-data display process is completed and thereby the entire biological-data measurement process is completed.

When the biological-data measurement process (FIG 4) advances to Step S19, a past-biological-data display process is executed.

In Step S 191, the timer is reset in the same manner as that in Step S161.

Then, in Step S 192, the display control of each of the biological data and the lighting control of the corresponding light emitter incorporated in the item key 9' are performed according to the first designation number X in the same manner as that in Step S162, except that the biological data to be displayed are the past biological data read in Step S 18, and the lighting mode (lighting pattern) of the light emitters 9 incorporated in the item keys 9' is determined by a third designation number Z instead of the second designation number Y. The third designation number Z is programmed in such a manner as to be initially set to "1" and incremented by one in the after-mentioned Step S 199, wherein the light emitters are blinked at a low speed (activated in a low-speed blinking mode) when the third designation number Z is "1", and blinked at a high speed (activated in a high-speed blinking mode) when the third designation number Z is "2". In this manner, the lighting mode is prepared differently between the latest-biological-data display and the past-biological-data display, and the designation number is set differently between the respective lighting modes. Thus, the lighting mode of the light emitters (item keys 9') during the latest-biological-data display can be changed from that during the past-biological-data display.

Then, in Step S193', it is determined whether one of the item keys 9' has been pressed down, and, if YES, the designation number Xinput of the pressed item key 9' is checked, in the same manner as that in Step S163'. If the determination in Step S 193 is NO or none of the item keys 9' has been pressed down, the process will advance to Step S 194. When the determination in Step S 193 is YES or either one of the item keys 9' has been pressed down, the process advances to Step S 197.

In Step S194, it is determined whether the third designation number Z has a value of "2". When the determination in Step S 194 is NO or the third designation number Y ≠ "2", the process advances to Step S195. If the determination in Step S194 is YES or the third designation number Y = "2", the process will skip Step S 195 and advance to Step S 196.

In Step S195, it is determined whether 3 × x seconds have elapsed from the timer reset in Step S191, in the same manner as that in Step S165. When the determination in Step S195 is YES or 3 × x seconds have elapsed, the process advances to Step S 196 through Step S200A. If the determination in Step S195 is NO or 3 × x seconds have not elapsed, the process will advance to Step S 196 without going through Step S200A.

In Step S196, it is determined whether 30 seconds have elapsed from the timer reset in Step S191 or the after-mentioned Step S197, in the same manner as that in Step S166. If the determination in Step S 196 is YES or 30 seconds have elapsed, the past-biological-data display process will be completed. When the determination in Step S196 is NO or 30 seconds have not elapsed, the process returns to Step S192.

In Step S197, the timer is reset in the same manner as that in Step S167, and then the process advances to Step S 198.

Then, in Step S 198, it is determined whether the third designation number Z has a value of "2". If the determination in Step S 198 is NO or the value of the second designation number Y ≠ "2", the process will advance to Step S 199. When the determination in Step S 198 is YES or the value of the second designation number Y = "2", the process skips Step S 199 and advances to Step S200B.

In Step S 199, the value of the third designation number Z is incremented by one or becomes "2". Thus, the light emitter 9 incorporated in the item key 9' is activated in the lighting mode defined by the third designation number "2" (blinked at a high speed).

In Step S200B, the first designation number X is changed depending on the designation number Xinput (either one of"1" to "9") assigned to the item key 9' pressed down in Step S163', in the same manner as that in Step S 170B, and then the process advances to Step S 196. When the determination in Step S196 is YES or 30 seconds have elapsed, the past-biological-data display process is completed and thereby the entire biological-data measurement process is completed.

FIG 10(A) to 10(D) illustrate the display of the liquid-crystal display screen 5 and the lighting mode of the light emitters incorporated in the item keys 9' in the apparatus 1'. The item keys 9' consisting of the nine key switches 91' to 99' are disposed adjacent to the liquid-crystal display screen 5. The key switches 91' to 99' are covered, respectively, with nine flexible light-transparent resin films 91a', 92a', 93a', 94a', 95a', 96a', 97a', 98a', 99a'. Each of the key switches 91' to 99' includes built-in electric contacts designed to be switched between contact and non-contact states in a conventional manner when it is pressed down, and a LED disposed inside each of the films 91a' to 99a' to serve as the light emitter. Further, each of the films 91a to 99a has a mark representing the biological datum associated therewith.

FIG 10(A) shows one example of a display about body weight (first designation number X = "1") as one of the biological data. A value "73.1" indicative of body weight and its unit "kg" are displayed on the liquid-crystal display screen 5, and the key switch 91' covered with the film 91a' having a mark representing body weight (weight mark) is lighted or activated in a normal lighting mode (second designation number Y = 1). That is, this screen currently displays a body weight datum which is a latest datum just after being measured, and will be automatically switched to display another biological datum (first designation number X = "2") after 3 seconds.

FIG 10(B) shows one example of a display about body fat percentage (first designation number X = "2") as another of the biological data. A value "21.7" indicative of body fat percentage and its unit "%" are displayed on the liquid-crystal display screen 5, and the key switch 92' covered with the film 92a' having a mark representing body fat percentage (mark having a white symbol "%" on a human-body-shaped colored background) is activated in the medium-speed blinking mode (second designation number Y = 2). That is, this screen currently displays a body fat percentage datum which is a latest datum just after being measured, and will be continuously displayed for 30 seconds unless the user presses either one of the item keys 9'.

FIG 10(C) shows one example of a display about bone mass (first designation number X = "8") as still another of the biological data. A value "4.7" indicative of bone mass and its unit "kg" are displayed on the liquid-crystal display screen 5, and the key switch 98' covered with the film 98a' having a mark representing bone mass (mark having while bone-shaped lines on a human-body-shaped colored background) is activated in the low-speed blinking mode (third designation number Z = "1"). That is, this screen currently displays a bone mass datum which is stored in a storage device 16 after being measured at a certain past time, and will be automatically switched to display another biological datum (first designation number X = "9") after at least 3 seconds.

FIG 10(D) shows one example of a display about visceral fat level (first designation number X = "9") as yet another of the biological data. A value "9" indicative of visceral fat level is displayed on the liquid-crystal display screen 5, and the key switch 99' covered with the film 99a' having a mark representing visceral fat level (mark having a white abdominal section on a human-body-shaped colored background) is activated in the high-speed blinking mode (third designation number Z = 2). That is, this screen currently displays a visceral fat level datum which is stored in the storage device 16 after being measured at a certain past time, and will be continuously displayed for 30 seconds unless the user presses either one of the up key 61 or the down key 62.

As described above, in the apparatus 1' according to the second embodiment, data measurement means primarily composed of the input device 6, the weight sensor 13, the current-supply electrodes 31, 32, the current-supply circuit 11, the measurement electrodes 41, 42, the voltage measurement circuit 12, and the control circuit 10, measures the plural (nine) kinds of biological data about a user, as in Steps S4 to S6 and Steps S 14 and S 15. Then, data selection means primarily composed of the item keys 9' and the control device 10 selects one of the measured biological data as in Steps S163' to S 170 or Steps S193' to S200, and data display means primarily composed of the liquid-crystal display screen 5 and the control device 10 displays the selected biological datum as in Step S162 or 5192. In particular, the plurality (nine) of light emitters primarily composed of the LEDs are incorporated in the corresponding key switches 91' to 99' in association, respectively, with the biological data, and lighting control means primarily composed of the control device 10 activates the light emitter incorporated in the item key 9' associated with the selected biological datum, as in Step S 162 or S192.

While the biological data measurement apparatus of the present invention has been described in connection with specific embodiments, the present invention is not limited to the specific embodiments, but various modifications and changes may be made therein without departing from the spirit and scope thereof as set forth in appended claims. For example, the number of kinds of the biological data is not limited to nine, but may be less than or greater than nine. The light emitter is not limited to an LED, but any other suitable type, such as a light bulb, may be used. Further, the mark in the light-transparent film covering each of the light emitters may be a character representing the associated biological datum. The change in lighting mode of the light emitters is not limited to switching between normal lighting and blinking, but may be any other suitable means, for example switching between different colors. It is also understood that the present invention may be widely applied to not only the so-called body composition analyzer utilizing bioelectric impedance but also any apparatus for measuring plural kinds of biological data (e.g. a pedometer designed to measure the number of steps, consumed energy and required energy).

## Claims

1. A biological data measurement apparatus including data measurement means for measuring plural kinds of biological data about a user, data selection means for selecting one of said plural kinds of measured biological data, and data display means for displaying said selected biological datum, said biological data measurement apparatus being **characterized by** comprising:
a plurality of light emitters corresponding, respectively, to said plural kinds of biological data; and
lighting control means for activating one of said light emitters which corresponds to one selected from said biological data by said data selection means.

2. The biological data measurement apparatus as defined in claim 1, wherein each of said light emitters includes a light-transparent film covering thereover and having a character or mark distinctively representing each biological datum associated therewith.

3. The biological data measurement apparatus as defined in claim 1 or 2, wherein said data selection means includes:
automatic data-selection means for automatically selecting each of the plural kinds of biological data one-by-one at given time intervals; and
arbitrary data-selection means for arbitrarily selecting one of the plural kinds of biological data according to a selection operation performed by the user,
wherein said lighting control means is operable to change a lighting mode of said light emitters depending on whether the selection of the biological data is performed by said automatic data-selection means or said arbitrary data-selection means.

4. The biological data measurement apparatus as defined in claim 1 or 2, which further comprises data storage means for storing the plural kinds of biological data measured by said data measurement means, wherein said data selection means includes:
latest-data selection means for selecting one of latest biological data newly measured by said data measurement means; and
past-data selection means for selecting one of past biological data stored on said data storage means,
wherein said lighting control means is operable to change a lighting mode of said light emitters depending on whether the selection of the biological data is performed by said latest-data selection means or said past-data selection means.

5. The biological data measurement apparatus as defined in either one of claims 1 to 4, wherein said data selection means includes a plurality of key switches associated, respectively, with said plural kinds of biological data, and each of said plurality of light emitters is incorporated in one of said key switches which is associated with the biological datum corresponding to said light emitter.

6. The biological data measurement apparatus as defined in either one of claims 1 to 5, wherein said data measurement means includes:
an input key for inputting at least a body height datum of the user;
a weight sensor for measuring a body weight datum of the user; and
a combination of an electrode and an electric circuit, for measuring a datum about an impedance between the feet of the user,
wherein said plural kinds of biological data consist of a plurality of index values about a body composition of the user to be calculated based on at least said body height, body weight and impedance data.
